# EUROPÄISCHE PATENTSCHRIFT

(11) **EP 1 109 046 B1**
(45) Veröffentlichungstag und Bekanntmachung des Hinweises auf die Patenterteilung: **25.09.2002**
(21) Anmeldenummer: 99125019.2
(22) Anmeldetag: 15.12.1999
(51) Int. Cl.: G02B 21/08

(54) **Beleuchtungseinrichtung für ein Operationsmikroskop**
Illumination system for an operation microscope
Dispositif d'éclairage pour un microscope opératoire

(43) Veröffentlichungstag der Anmeldung: 20.06.2001
(73) Patentinhaber: Möller-Wedel GmbH, 22880 Wedel (DE)
(72) Erfinder: Koetke, Jochen, Dr., 22083 Hamburg (DE)
(74) Vertreter: Glawe, Delfs, Moll & Partner

(56) Entgegenhaltungen:
- DE-A- 3 623 613
- DE-A- 19 650 773
- FR-A- 2 666 662
- US-A- 4 783 159

## Beschreibung

Die Erfindung betrifft eine Beleuchtungseinrichtung für ein Operationsmikroskop mit einer Lichtquelle, mit der das beobachtete Objekt durch einen außerhalb der optischen Achse liegenden Bereich des Mikroskopobjektivs beleuchtbar ist, und mit zwei senkrecht zur optischen Achse verschiebbaren Reflexionselementen, von denen das erste einen Teil des Lichts in eine zur optischen Achse senkrechte Richtung umlenkt und das zweite dieses Lichts in einen achsnahen Bereich umgelenkt.

Beleuchtungseinrichtungen für Operationsmikroskope verwenden meistens einen Lichtweg für die Beleuchtung, der mit dem Beobachtungsstrahlengang nur einen kleinen Winkel häufig im Bereich von ca. 6° bildet. Dies ist z. B. wichtig, wenn man bei einer Operation tieferliegende Bereiche betrachten möchte. Wenn die Beleuchtung nicht ungefähr parallel zum Beobachtungsstrahlengang ist, so würde der zu beobachtende Bereich im Schatten liegen.

Augenchirurgische Operationen stellen besondere Anforderungen an die Beleuchtung. Wichtig ist der Winkel, unter dem das Auge relativ zum Beobachtungsstrahlengang des Operateurs beleuchtet wird. Eine gute Plastizität des Bildes durch die Schattenbildung an Strukturen im Augeninneren wird bei der Beleuchtung des Auges unter einem Winkel von einigen Grad, häufig um 6° zum Beobachtungsstrahlengang erzielt. Wird das Auge dagegen möglichst koaxial zum Beobachtungsstrahlengang beleuchtet (d.h., der Winkel zwischen Beobachtungsstrahlengang und Beleuchtungsstrahlengang ist möglichst gering), führt dieses zur Ausbildung des sogenannten Rotreflexes. Die Pupille des operierten Auges leuchtet durch das von der Netzhaut zurückgestreute Licht rötlich auf. Diese Beleuchtungsart ist bei Katarakt-Operationen sehr vorteilhaft, denn Gewebereste, die beim Entfernen der Linse anfallen und zur Vermeidung von Komplikationen unbedingt zu entfernen sind, lassen sich im Gegenlicht des Rotreflexes gut erkennen. Die Rotreflexerzeugung ist zu einem wichtigen Hilfsmittel moderner Operationstechniken geworden.

Weil bei den zur Rotreflexerzeugung benötigten geringen Winkeln zwischen den Beleuchtungs- und Beobachtungsstrahlengängen die erwähnte Plastizität des Bildes nicht zu erzielen ist, ist zusätzlich zur Rotreflexbeleuchtung die gleichzeitige Beleuchtung unter ca. 6° vorteilhaft. Nicht in allen Stadien der Operation ist aber ein Rotreflex erwünscht. Ein optimales Beleuchtungsmodul muß daher zwei Beleuchtungseinstellungen bieten: In der ersten Stellung wird das OP-Feld mit einer Kombination aus der 6°-Beleuchtung und der koaxialeren Beleuchtung zur Rotreflexerzeugung beleuchtet. In der zweiten Stellung wird ausschließlich unter 6° beleuchtet.

Es ist bekannt, einen Teil des achsfernen Lichtes umzulenken und zur achsnahen Beleuchtung zu verwenden (US-A 4,779,968). Die entsprechenden Umlenkeinrichtungen sind aber unterhalb des Mikroskopobjektivs angebracht, was den Manövrierabstand unterhalb des Mikroskops verringert. Es wird dort auch im konvergenten Strahlengang ein Strahlenteiler verwendet, der durch den Beobachtungsstrahlengang verläuft, was die optische Qualität des Mikroskops beeinträchtigt.

Es ist bekannt, das von der Beleuchtung kommende Licht aufzuteilen, wobei ein erster Spiegel, der das achsferne Licht erzeugt, einen Teil des Lichts auf einen zweiten, achsnahen Spiegel für achsnahe Beleuchtung passieren läßt (DE 40 28 605 A1). Dieser zweite Spiegel ist senkrecht zur optischen Achse des Mikroskopobjektivs verschiebbar, so daß der Winkel variiert werden kann, den das achsnahe Beleuchtungslicht mit der optischen Achse des Mikroskopobjektivs bildet. Diese Anordnung ist jedoch nicht vollständig aus dem Strahlengang herausschaltbar. Durch die Umlenkung mit mindestens zwei getrennten Umlenkspiegeln führt dies zur unsauberer Abbildung des Präzisions-Beleuchtungsspalts einer Mikroskop-Spaltlampe. Es entstehen Doppelbilder.

Bei einer weiteren vorbekannten Beleuchtungseinrichtung für ein Operationsmikroskop (US-A-5,760,952) fällt das senkrecht zur optischen Achse des Mikroskopobjektivs einfallende Licht auf zwei Umklenkspiegel, durch die der Beleuchtungswinkel der achsfernen Beleuchtung variiert werden kann und die Menge des Lichtes beeinflußt werden kann, der auf einen weiteren Umlenkspiegel für die nachsnahe Beleuchtung fällt. Der Winkel der achsnahen Beleuchtung kann aber nicht variiert werden.

Bei einer Beleuchtungseinrichtung der eingangs genannten Art (US-A-4,783,159) können beide Refelexionseinrichtungen gemeinsam verschoben werden, so daß der Beleuchtungswinkel des achsnahen Strahls variiert werden kann. Soll auf die achsnahe Beleuchtung verzichtet werden, so müssen die Reflexionselemente aus dem Strahlengang entfernt werden. Wünscht der Operateur häufiger von achsnaher Beleuchtung für den Rotreflex auf die kontrastreichere Beleuchtung mit achsfernen Strahlen umzuschalten, so ist für die Erzielung des Rotreflexes jeweils erneute Justierung der Reflexionselemente erforderlich.

Die Aufgabe der Erfindung besteht in der Schaffung einer Beleuchtungseinrichtung der eingangs genannten Art, die größere Variabilität besitzt und mit der die achsnahe Beleuchtung ausgeschaltet werden kann, ohne die Stellung des entsprechenden Reflexionselementes verändern zu müssen.

Die erfindungsgemäße Lösung besteht bei einer Beleuchtungseinrichtung der eingangs genannten Art darin, daß die Reflexionselemente unabhängig voneinander verschiebbar sind.

Durch Verschieben des ersten Reflexionselementes kann die Lichtmenge verändert werden, die auf das zweite Reflexionselement fällt. Es kann dadurch ein mehr oder weniger starker Rotreflex erzeugt werden. Ist das erste Reflexionselement vollständig aus dem Strahlengang der achsfernen Beleuchtung herausgeschoben, so wird überhaupt kein achsnahes Licht für den Rotreflex zum Objekt geleitet. Wird das erste Reflexionselement von außen langsam in den Strahlengang der achsfernen Beleuchtung geschoben, so wird der Rotreflex verstärkt. Gleichzeitig werden die achsferneren Bereiche der achsfernen Beleuchtung abgedeckt, so daß sich auch der Winkel des noch eingestrahlten achsfernen Lichtes mit der optischen Achse des Mikroskopobjektivs verringert. Soll zwischenzeitlich auf den Rotreflex verzichtet werden, so wird das erste Reflexionselement aus dem Strahlengang verschoben. Soll anschließend wieder der Rotreflex erzeugt werden, so wird dies nach Wiedereinführen des ersten Reflexionselementes in den Strahlengang mit demselben Winkel für die achsnahe Beleuchtung erzielt, da die Stellung des zweiten Reflexionselementes für die Ausschaltung des Rotreflexes nicht verändert worden ist.

Da das zweite Reflexionselement verschiebbar ist, kann der Winkel für die achsnahe Beleuchtung und damit die Qualität des Rotreflexes abhängig von der Einstellung des Vergrößerungsfaktors des Mikroskops durch Zoom oder Galilei-Wechsler verbessert werden. Bei höheren Vergrößerungsfaktoren kann dieses Reflexionselement vom Anwender nahe den Beobachtungsstrahlengängen oder sogar überlappend mit diesen plaziert werden (wodurch ein intensiverer Rotreflex erzeugt wird), ohne daß eine störende Vignettierung erkennbar wird. Ist das Reflexionselement bei kleinerer Vergrößerung mit dem Beobachtungsstrahlengang überlappend eingestellt, kann es zur Vignettierung kommen. In dem vom Anwender wahrgenommenen Mikroskopbild kommt es stellenweise zur Abdunkelungen, die von vielen Anwendern als störend empfunden werden. Selbstverständlich kann das zweite Reflexionselement unter Inkaufnahme einer Vignettierung für einen verbesserten Rotreflex auch bei niedrigen Vergrößerungen soweit in die Strahlengänge verschoben werden, daß Vignettierung sichtbar wird. Der Anwender kann die für die konkrete Situation beste Wahl treffen.

Die Anordnung kann auch im Gegensatz zur vorbekannten Anordnungen so getroffen werden, daß das erste Reflexionselement bei Nichtgebrauch völlig aus dem Beleuchtungsstrahlengang herausbewegt wird. Nur durch die vollständige Entfernung kann höchste Abbildungsqualität für den Einsatz einer Operationsspaltlampe gewährleistet werden, die einen feinen Lichtspalt in die Beobachtungsebene abbildet. Bei der vorbekannten Beleuchtungseinrichtung (US-A-5,760,952) ist für die Abbildung eines feinen Lichtspalts der Spaltlampe bei ausgeschaltetem Rotreflexverstärker eine sehr präzise Justierung zweier Spiegel zueinander erforderlich, von denen einer beweglich ist. Dies ist in der Praxis mit vernünftigem Aufwand nicht erfüllbar, so daß Qualitätseinbußen bezüglich der Abbildung in Kauf genommen werden müssen. Für die erfindungsgemäße Beleuchtungseinrichtung ist die Justierung deutlich unkritischer, weil für die Rotreflexverstärkung wesentlich geringere Anforderungen an die Abbildungsgenauigkeit erforderlich sind und die Beleuchtungseinrichtung für Spaltlampenanwendungen vollständig aus dem Beleuchtungsstrahlengang herausschaltbar ist.

Der Beleuchtungswinkel kann also für das gesamte oder auch nur für einen Teil des Lichts verändert werden. Bedarf für diese Möglichkeit besteht in der Ophtalmologie und generell dann, wenn der Beleuchtungswinkel der jeweiligen Operationssituation angepaßt werden soll. In der Ophtalmologie ist in vielen Fällen das Erzeugen des Rotreflexes oder Fundusreflexes für sicheres Arbeiten notwendig. In anderen Situationen kann es günstig sein, den Beleuchtungswinkel zu vergrößern, um durch stärkeren Schattenwurf der beobachteten Strukturen die Plastizität des wahrgenommenen Bildes zu erhöhen. All dies ist mit der erfindungsgemäßen Beleuchtungseinrichtung möglich.

Durch die Veränderung der Position des ersten Reflexionselementes kann z. B. der Anteil mit geringem Winkel zum Beobachtungsstrahlengang oder mit großem Winkel zum Beobachtungsstrahlengang ausgewählt werden. In der Ophtalmologie kann das erste Reflexionselement so positioniert werden, daß die Reflexion der achsfernen Beleuchtung an der Cornea des Patienten dem Operateur als ein Reflex erscheint. In anderen Disziplinen ist es vorteilhaft, daß erste Reflexionselement mittig in die achsferne Beleuchtung zu positionieren, weil dann die Lichtmenge, die auf das zweite Reflexionselement gelenkt wird, maximal ist.

Die optische Achse der Rotreflexbeleuchtung fällt bei der erfindungsgemäßen Beleuchtungseinrichtung im Gegensatz zu vorbekannten Beleuchtungseinrichtungen bewußt nicht mit der optischen Achse des Mikroskopobjektivs und den Beobachtungsstrahlengängen zusammen. Die absolute Koinzidenz führt nämlich zu einem "flauen Bild", das ungenügende Plastizität besitzt. Die Spiegelbreite ist außerdem bei absoluter Koinzidenz auf kleine Werte begrenzt, weil sonst die Beobachtungsstrahlengänge abgedeckt werden. Dadurch ist die Lichtmenge für die meisten Operationsabschnitte nicht ausreichend: "Polieren" der hinteren Linsenkapsel, Phakoe-mulsifikation.

Bei einer vorteilhaften Ausführungsform sind die beiden Reflexionselemente und der Verschiebungsmechanismus in einem entfernbaren Modul angeordnet. Das Modul wird zwischen dem Mikroskopobjektiv und der mikroskopeigenen achsfernen Beleuchtung installiert, die das Licht der Beleuchtungsquelle zum Mikroskopobjektiv lenkt. Vor und hinter dem Modul können Zubehörteile des Mikroskops, z. B. Assistenmikroskope installiert werden. Das Modul kann direkt hinter dem Mikroskopobjektiv oder aber auch in beliebiger Reihenfolge mit anderen Modularenzubehörteilen installiert werden, z. B. direkt vor der achsfernen Beleuchtung. Vorteilhaft ist die Positionierung direkt hinter dem Mikroskopobjektiv, um das Einstreuen von Reflexen der Beleuchtung in den Beobachtungsstrahlengang gering zu halten. Auch ein Nachrüsten beim Kunden ist problemlos möglich.

Bei einer vorteilhaften Ausführungsform ist das erste Reflexionselement auf seiner von der optischen Achse wegzeigenden Seite mit einer Blende versehen. Dadurch kann achsferneres Licht abgedeckt werden, wenn das Reflexionselement näher zur optischen Achse des Mikroskopobjektivs verschoben wird. Es kann aber auch vorgesehen sein, daß die Blende teilweise Lichtdurchlässig ist, so daß ein schwacher Anteil des achsferneren Lichtes noch zum beleuchtenden Objekt gelangen kann.

Es können Maßnahmen getroffen werden, daß das erste und/oder zweite Reflexionselement kurzwellige Anteile des reflektierten Lichts nur geschwächt weiterleitet, indem es z. B. mit einem Filter oder einer entsprechenden Beschichtung versehen ist. Ist das Reflexionselement ein Prisma, so könnte dieses aus einem Material hergestellt sein, das beim Durchgang des Lichtes den kurzwelligen Anteil abschwächt. Außer Prismen oder zusätzlich zu Prismen können als Reflexionselemente auch Spiegel verwendet werden.

Vorteilhafterweise sind die Reflexionselemente mit Hilfe von koaxialen Drehknöpfen verschiebbar.

Zweckmäßigerweise ist die zu den Beobachtungsstrahlengängen gerichtete Begrenzung der zweiten Reflexionselemente parallel zu der Tangente des Umfangs dieser Strahlengänge, an der die Reflexionselemente an diesen Strahlengang herankommen. Diese Begrenzung kann aber auch so ausgebildet sein, daß sie die Strahlengänge dann teilkreisförmig umgreift.

Die Erfindung wird im folgenden anhand einer vorteilhaften Ausführungsform unter Bezugnahme auf die beigefügten Zeichnungen beschrieben. Es zeigen:
- Fig. 1 und 2: in schematischer Darstellung die Wirkungsweis der erfindungsgemäßen Beleuchtungseinrichtung bei verschiedenen Stellungen der Reflexionselemente;
- Fig. 3: einen Schnitt durch den wesentlichen Teil der Beleuchtungseinrichtung von der Seite gesehen;
- Fig. 4: eine Ansicht der wesentlichen Teile von unten; und
- Fig. 5: eine entsprechende Ansicht der wesentlichen Teile der Beleuchtungseinrichtung von oben.

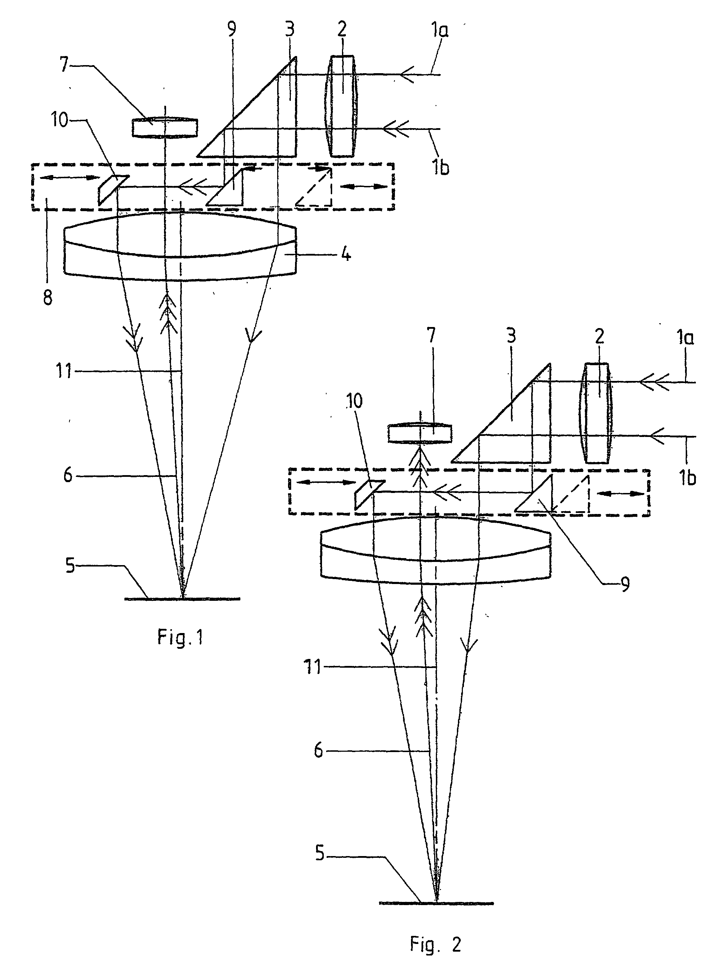

Wie dies in den Figuren 1 und 2 gezeigt ist, gelangen mit einem bzw. zwei Pfeilen bezeichnete Lichtstrahlen la und 1b von einer nicht gezeigten Lichtquelle durch eine Linse 2 zu einem Umlenkprisma 3, das den achsfernen Strahl la durch die Objektivlinse 4 auf das Beobachtungsobjekt 5 lenkt. Die Beobachtung erfolgt dann auf dem Wege des Strahles 6 durch eine Linse 7 und weitere bei einem Mikroskop übliche optische Elemente, die in diesen Darstellungen nicht gezeigt sind. Unmittelbar hinter dem Objektiv 4 befindet sich ein bei 8 gestrichelt angedeutetes Modul mit zwei Reflexionselementen in Form von Spiegeln. Sowohl das erste rechts angeordnete Reflexionselement 9 auch als das zweite links angeordnete Reflexionselement 10 können, wie dies durch Pfeile gezeigt ist, verschoben werden. Befindet sich der erste Spiegel 9 in der in Figur 1 gestrichelten Position, so wird das gesamte Licht la, 1b achsfern auf das Objekt 5 gerichtet. Befindet sich der Spiegel 9 in der in Figur 1 ausgezogen gezeichneten Stellung, so wird ein Teil des Lichtes, nämlich 1b auf den zweiten Spiegel 10 gelenkt und von dort unter einem kleineren Winkel relativ zur optischen Achse 11 des Mikroskopobjektivs 4 auf das Objekt 5 gerichtet.

Der Unterschied der Stellung der Spiegel 9, 10 in Figur 2 zur Figur 1 besteht darin, daß der Spiegel 9 achsferneres Licht la auf den zweiten Spiegel 10 lenkt und achsnäheres Licht 1b zusätzlich zur Beleuchtung dient. Man hat hier den Fall insgesamt kleiner Winkel der Beleuchtung, was den Rotreflex erhöht.

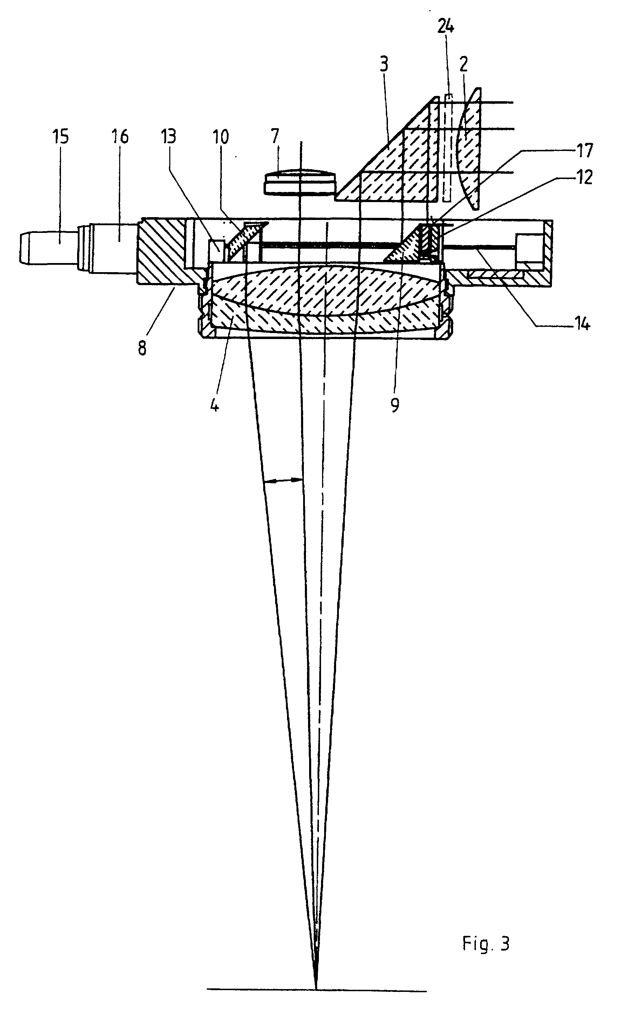

In Figur 3 ist das hier modulartig ausgebildete Gehäuse 8 mit den beiden verschiebbaren Spiegeln 9, 10 im größeren Detail gezeigt. Diese Spiegel sind auf Gleitern 12, 13 montiert, die auf Schienen 14 gleiten können. Die Verstellung der beiden Spiegel 9, 10 erfolgt dabei unabhängig voneinander durch die koaxialen Drehknöpfe 15, 16. Eine Blende 17 zur Abdeckung von Licht ist hinter dem Spiegel 9 angeordnet. Bei 24 ist gestrichelt ein Filter angedeutet, mit dem der kurzwellige Anteil des Lichts abgeschwächt wird.

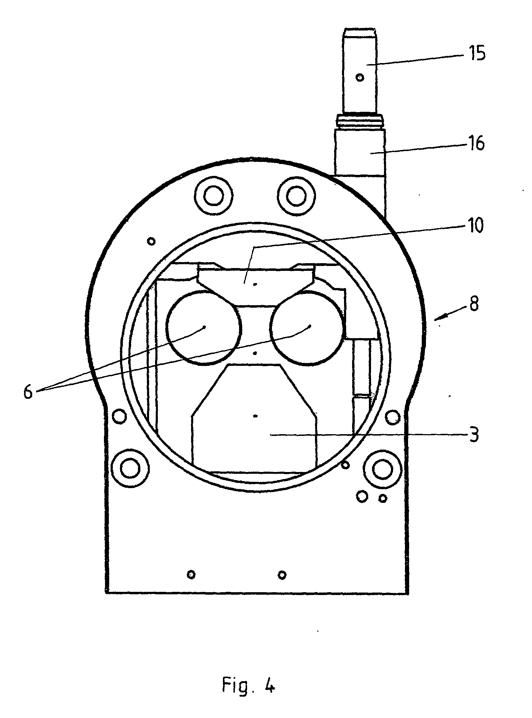

Figur 4 zeigt die wesentlichen Teile der erfindungsgemäßen Beleuchtungseinrichtung von unten. Man erkennt dort, daß der zweite Spiegel 10 die beiden Beobachtungsstrahlengänge 6 des Stereomikroskops gerade berührt. Seine Begrenzungsfläche ist dabei tangential zum Umfang der Beobachtungsstrahlengänge 6, könnte aber auch mit teilkreisförmigen Ausschnitten versehen sein und die Strahlengänge 6 teilweise umgreifen (nicht gezeigt). Zur Veränderung des Winkels für den Rotreflex kann dieser Spiegel aber auch wie erwähnt näher an die Beobachtungsstrahlengänge oder weiter weg von demselben bewegt werden.

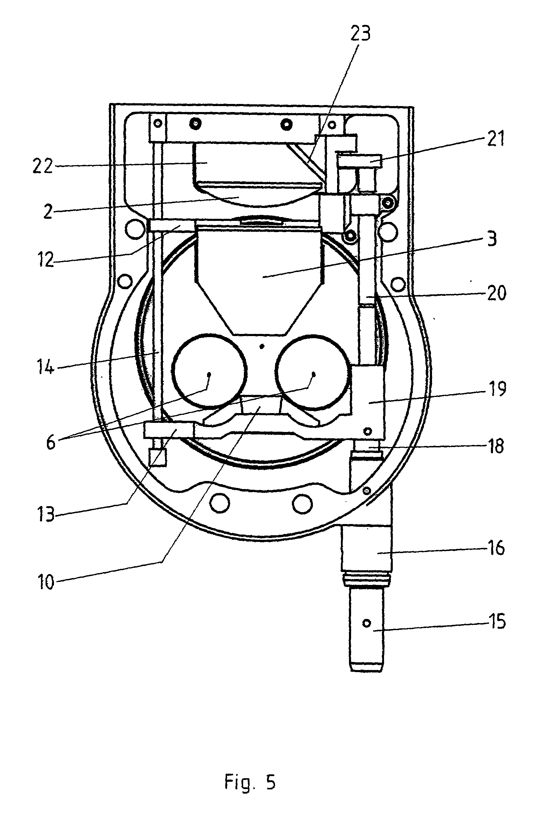

Figur 5 zeigt das Modul 8 von oben. Der Rotationsknopf 16 ist mit einer Gewindestange 18 verbunden, die durch eine Mutter 19 hindurchgeht, die am Gleiter 13 angeordnet ist. Wird der Rotationsknopf 16 gedreht, so wird dadurch der Gleiter 13 mit dem zweiten Spiegel 10 in die eine oder andere Richtung bewegt. Durch das Element 16 hindurch ist eine Achse 20 koaxial hindurchgeführt, die mit dem Drehknopf 15 verbunden ist. Am Ende dieser Achse 20 befindet sich ein Exzenter 21, durch den eine Platte 22 nach rechts oder links bewegt werden kann. Diese Platte weist eine Nut 23 auf, in die ein Stift des Gleiters 12 hineinfaßt. Wird die Platte 23 durch Drehen des Knopfes 15 in der Figur nach rechts und links bewegt, so bewegt sich der Gleiter 12 mit dem darauf angeordneten (in Figur 5 durch das Prisma 3 verdeckten) Spiegel 9 in die eine oder andere Richtung. Durch diese Exzentersteuerung kann der Spiegel 9 durch verhältnismäßig wenig Drehung am Drehknopf 15 sehr stark und schnell verstellt werden.

## Patentansprüche

1. Beleuchtungseinrichtung für ein Operationsmikroskop mit einer Lichtquelle, mit der das beobachtete Objekt durch einen außerhalb der optischen Achse liegenden Bereich des Mikroskopobjektivs beleuchtbar ist, und mit zwei senkrecht zur optischen Achse verschiebbaren Reflexionselementen, von denen das erste einen Teil des Lichts in eine zur optischen Achse senkrechte Richtung umlenkt und das zweite dieses Licht in einen achsnahen Winkelbereich zum Objekt hin umlenkt, **dadurch gekennzeichnet, daß** die Reflexionselemente (9, 10) unabhängig voneinander verschiebbar sind.

2. Beleuchtungseinrichtung nach Anspruch 1, **dadurch gekennzeichnet, daß** die beiden Reflexionselemente (9, 10) und der Verschiebungsmechanismus (12-16, 18-23) in einem entfernbaren Modul (8) angeordnet sind.

3. Beleuchtungseinrichtung nach Anspruch 1 oder 2, **dadurch gekennzeichnet, daß** das erste Reflexionselement (9) auf seiner von der optischen Achse (11) wegzeigenden Seite mit einer Blende (17) versehen ist.

4. Beleuchtungseinrichtung nach Anspruch 3, **dadurch gekennzeichnet, daß** die Blende (17) lichtundurchlässig ist.

5. Beleuchtungseinrichtung nach Anspruch 3, **dadurch gekennzeichnet, daß** die Blende (17) teildurchlässig für Licht ist.

6. Beleuchtungseinrichtung nach einem der Ansprüche 1-5, **dadurch gekennzeichnet, daß** das erste und/oder zweite Reflexionselement (9, 10) mit einem Filter (24) zur Schwächung des kurzwelligen Anteils des reflektierten Lichts versehen ist.

7. Beleuchtungseinrichtung nach einem der Ansprüche 1-5, **dadurch gekennzeichnet, daß** das erste und/oder zweite Reflexionselement (9, 10) mit einer Beschichtung zur Schwächung des kurzwelligen Anteils des reflektierten Lichts versehen ist.

8. Beleuchtungseinrichtung nach einem der Ansprüche 1-7, **dadurch gekennzeichnet, daß** die Reflexionselemente mit Hilfe von koaxialen Drehknöpfen (15, 16) verschiebbar sind.

9. Beleuchtungseinrichtung nach einem der Ansprüche 1-8, **dadurch gekennzeichnet, daß** die Reflexionselemente (9, 10) Spiegel aufweisen.

10. Beleuchtungseinrichtung nach einem der Ansprüche 1-9, **dadurch gekennzeichnet, daß** die Reflexionselemente (9, 10) Prismen aufweisen.

11. Beleuchtungseinrichtung nach einem der Ansprüche 1-10, **dadurch gekennzeichnet, daß** der zweite Spiegel (10) zu den Beobachtungsstrahlengängen (6) eine Begrenzung aufweist, die zu diesem tangential ist.

12. Operationsmikroskop, **dadurch gekennzeichnet, daß** es eine Beleuchtungseinrichtung nach einem der Ansprüche 1-11 aufweist.

## Claims

1. Illumination system for an operation microscope with a light source with which
the object observed can be illuminated through a region of the microscope objective lying outside the optical axis, and with two reflector elements movable at right angles to the optical axis, the first of which deflects a part of the light into a direction at right angles to the optical axis and the second deflects this light into a sector close to the axis to the object, **characterized by** the reflector elements (9, 10) being movable independently of each other.

2. Illumination system according to Claim 1, **characterized by** the two reflector elements (9, 10) and the displacement mechanism (12-16, 18-23) being arranged in a removable module (8).

3. Illumination system according to Claim 1 or 2, **characterized by** the first reflector element (9) being fitted with a diaphragm (17) on its side pointing away from the optical axis (11).

4. Illumination system according to Claim 3, **characterized by** the diaphragm (17) being opaque.

5. Illumination system according to Claim 3, **characterized by** the diaphragm (17) being partially transparent for light.

6. Illumination system according to one of Claims 1-5, **characterized by** the first and/or second reflector element (9, 10) being provided with a filter (24) to reduce the short-wave portion of the reflected light.

7. Illumination system according to one of Claims 1-5, **characterized by** the first and/or second reflector element (9, 10) being provided with a coating to reduce the short-wave portion of the reflected light.

8. Illumination system according to one of Claims 1-7, **characterized by** the reflector elements being movable with the aid of coaxial rotary knobs (15, 16).

9. Illumination system according to one of Claims 1-8, **characterized by** the reflector elements (9, 10) having mirrors.

10. Illumination system according to one of Claims 1-9, **characterized by** the reflector elements (9, 10) having prisms.

11. Illumination system according to one of Claims 1-10, **characterized by** the second mirror (10) having a boundary to the observation light paths (6) which is tangential to this.

12. Operation microscope **characterized by** having an illumination system according to one of Claims 1-11.

## Revendications

1. Dispositif d'éclairage pour un microscope chirurgical comportant une source de lumière au moyen de laquelle l'objet observé peut être éclairé à travers une zone de l'objectif du microscope située à l'extérieur de l'axe optique, et comportant deux éléments de réflexion qui peuvent coulisser perpendiculairement à l'axe optique, dont le premier dévie une partie de la lumière dans une direction perpendiculaire à l'axe optique, et le deuxième dévie cette lumière dans une plage angulaire voisine de l'axe, vers l'objet, **caractérisé en ce que** les éléments de réflexion (9, 10) peuvent coulisser indépendamment l'un de l'autre.

2. Dispositif d'éclairage selon la revendication 1, **caractérisé en ce que** les deux éléments de réflexion (9, 10) et le mécanisme de coulissement (12-16, 18-23) sont disposés dans un module (8) amovible.

3. Dispositif d'éclairage selon la revendication 1 ou 2, **caractérisé en ce que** le premier élément de réflexion (9) est pourvu d'un diaphragme (17) sur son côté opposé à l'axe optique (11).

4. Dispositif d'éclairage selon la revendication 3, **caractérisé en ce que** le diaphragme (17) ne laisse pas passer la lumière.

5. Dispositif d'éclairage selon la revendication 3, **caractérisé en ce que** le diaphragme (17) laisse en partie passer la lumière.

6. Dispositif d'éclairage selon l'une des revendications 1 à 5, **caractérisé en ce que** le premier et/ou le deuxième élément de réflexion (9, 10) sont pourvus d'un filtre (24) pour affaiblir la fraction à ondes courtes de la lumière réfléchie.

7. Dispositif d'éclairage selon l'une des revendications 1 à 5, **caractérisé en ce que** le premier et/ou le deuxième élément de réflexion (9, 10) sont pourvus d'un revêtement destiné à affaiblir la fraction à ondes courtes de la lumière réfléchie.

8. Dispositif d'éclairage selon l'une des revendications 1 à 7, **caractérisé en ce que** les éléments de réflexion peuvent coulisser à l'aide de boutons tournants (15, 16) coaxiaux.

9. Dispositif d'éclairage selon l'une des revendications 1 à 8, **caractérisé en ce que** les éléments de réflexion (9, 10) comportent des miroirs.

10. Dispositif d'éclairage selon l'une des revendications 1 à 9, **caractérisé en ce que** les éléments de réflexion (9, 10) comportent des prismes.

11. Dispositif d'éclairage selon l'une des revendications 1 à 10, **caractérisé en ce que** le deuxième miroir (10) comporte un organe de limitation des parcours (6) des rayons d'observation, qui est tangentiel à ce miroir.

12. Microscope chirurgical, **caractérisé en ce qu'**il comporte un dispositif d'éclairage selon l'une des revendications 1 à 11.
